# FASCICULE DE BREVET EUROPEEN

(11) **EP 3 054 962 B1**
(45) Date de publication et mention de la délivrance du brevet: **31.01.2018**
(21) Numéro de dépôt: 14798961.0
(22) Date de dépôt: 07.10.2014
(51) Int. Cl.: A61K 35/76, A61K 35/761, A61K 38/17, C12N 15/86

(54) **VECTEUR D'EXPRESSION DE LA PROTÉINE PUMA ET SON UTILISATION EN THÉRAPIE GÉNIQUE**
PUMA-PROTEIN-EXPRESSIONSVEKTOR UND VERWENDUNG DAVON IN DER GENTHERAPIE
PUMA PROTEIN EXPRESSION VECTOR AND USE THEREOF IN GENE THERAPY

(30) Priorité: 08.10.2013 FR 1359751
(43) Date de publication de la demande: 17.08.2016
(73) Titulaire: Hospices Civils de Lyon, 69002 Lyon (FR); The Regents of the University of California, Oakland, CA 94607-5200 (US); Université Claude Bernard Lyon 1, 69622 Villeurbanne Cedex (FR); Institut d'Enseignement Supérieur et de Recherche en Alimentation, Santé Animale, Sciences Agronomiques et de l'Environnement, 69280 Marcy l'Etoile (FR); Institut National de la Santé et de la Recherche Médicale (INSERM), 75013 Paris (FR)
(72) Inventeur: MIOSSEC, Pierre, 69500 Bron (FR); HONG, Saw-See, 69006 Lyon (FR); FIRESTEIN, Gary, Del Mar, CA 92104-3903 (US)
(74) Mandataire: Gevers & Orès
(86) Numéro de dépôt international: PCT/IB2014/065118
(87) Numéro de publication internationale: WO 2015/052651

(56) Documents cités:
- WO-A1-2011/042769
- WO-A2-2007/035494
- MYEW-LING TOH ET AL: "Enhancement of adenovirus-mediated gene delivery to rheumatoid arthritis synoviocytes and synovium by fiber modifications: role of arginine-glycine-aspartic acid (RGD)- and non-RGD-binding integrins", THE JOURNAL OF IMMUNOLOGY, vol. 175, no. 11, décembre 2005 (2005-12), page 7687, XP055096844, ISSN: 0022-1767
- WAFA KAMMOUNI ET AL: "Regulation of apoptosis in fibroblast-like synoviocytes by the hypoxia-induced Bcl-2 family member Bcl-2/adenovirus E1B 19-kd protein-interacting protein 3", ARTHRITIS & RHEUMATISM, vol. 56, no. 9, septembre 2007 (2007-09), pages 2854-2863, XP055096881, ISSN: 0004-3591, DOI: 10.1002/art.22853 cité dans la demande
- NIEDERMEIER M ET AL: "Therapeutic opportunities in fibroblasts in inflammatory arthritis", BAILLIERE'S BEST PRACTICE AND RESEARCH. CLINICAL REUMATOLOGY, BAILLIERE TINDALL, LONDON, GB, vol. 24, no. 4, août 2010 (2010-08), pages 527-540, XP027224439, ISSN: 1521-6942 [extrait le 2010-08-21]
- CHARLES J. MALEMUD: "Apoptosis Resistance in Rheumatoid Arthritis Synovial Tissue", JOURNAL OF CLINICAL & CELLULAR IMMUNOLOGY, vol. 01, no. S3, 21 décembre 2011 (2011-12-21), XP055096884, DOI: 10.4172/2155-9899.S3-006

## Description

La présente invention concerne un vecteur viral d'expression de la protéine PUMA (pour « *p53 up-regulated modulator of apoptosis* »), inductrice de mort cellulaire, pour son utilisation en thérapie génique, en particulier de la polyarthrite rhumatoïde.

La polyarthrite rhumatoïde (PR) est une maladie autoimmune qui entraîne une inflammation chronique des articulations et des tissus environnants. L'inflammation est entretenue par des cytokines produites par les macrophages synoviaux et les fibroblastes, telles que le facteur alpha de nécrose tumorale (TNF-α) et l'interleukine 1 (IL-1), et par d'autres cellules comme les lymphocytes T, telle que l'interleukine 17 (IL-17). La prolifération des synoviocytes fibroblastiques (FLS, pour *« fibroblast-like synoviocytes* »), ou synoviocytes de type B (ci-après désignés « synoviocytes »), contribue au processus inflammatoire par accumulation de cellules dans la couche intimale de la synoviale (Firestein, Arthritis Rheum., 39:1781-90, 1996). En effet, alors que dans une articulation saine, les synoviocytes participent à l'équilibre biochimique par la sécrétion de différentes protéines et cytokines, dans une articulation arthritique, ils subissent des modifications se traduisant par leur prolifération, la libération de protéines anti-apoptotiques, de cytokines proinflammatoires et d'enzymes protéolytiques, telles que des collagénases.

Les traitements actuels de la PR impliquent des biothérapies en particulier anti-TNF. Cependant, 40% des patients ne répondent pas au traitement ou y répondent par un effet thérapeutique transitoire. Il a été suggéré que les traitements locaux impliquant l'induction de l'apoptose des synoviocytes pourraient représenter un traitement efficace de la PR (Firestein, Nature, 423 :356-61, 2003).

A ce titre, plusieurs gènes codant pour des protéines pro-apoptotiques ont été évalués en tant que gènes thérapeutiques potentiels, par exemple les gènes codant pour les protéines Fas (Okamoto et al., Gene Ther., 5 :331-8, 1998), TRAIL (pour « *tumor necrosis factor-related apoptosis-inducing ligand* ») (Yao et al., Arthr. Res. Ther., 8 :IR16, 2006), p53 (Yao et al., Mol. Ther., 3(6) :901-10, 2001).

Parmi ces protéines pro-apoptotiques, on citera notamment les protéines *« BH3-only* » de la famille Bcl-2. La Demande PCT WO 2007/035494 propose l'utilisation de ces protéines pour induire l'apoptose dans les synoviocytes. Des résultats préliminaires ont montré que la transduction *in vitro* de synoviocytes avec un vecteur plasmidique exprimant la protéine PUMA (PCT WO 2007/035494 ; Cha et al., Arthritis & Rheumatism, 54(2) :587-92, 2006 ; You et al., 2006, précité) permettait d'induire l'apoptose des cellules transduites. D'autres expérimentations du même type ont montré que la protéine *« BH3-only* » BNIP3 possédait les mêmes propriétés pro-apoptotiques, mais des essais complémentaires effectués en présence de cytokines proinflammatoires, telles que TNF-α et IL-1β, ont montré que cet effet pro-apoptotique était partiellement inhibé par ces cytokines proinflammatoires, qui sont présentes abondamment dans les articulations touchées par la PR (Kammouni et al., Arthritis & Rheum., 56(9) :2854-63, 2007).

Les Inventeurs ont entrepris d'étudier de manière plus approfondie les effets de la protéine PUMA sur l'apoptose des synoviocytes dans un contexte inflammatoire, afin de déterminer si l'utilisation de cette protéine *in vivo* dans le cadre du traitement de la PR était ou non effectivement envisageable. Dans ce but, ils ont dans un premier temps, pour obtenir une expression efficace *in vivo,* cherché une alternative aux vecteurs plasmidiques utilisés dans l'art antérieur.

Les adénovirus recombinants sont parmi les vecteurs les plus utilisés *in vivo,* pour l'expression de gènes à but thérapeutique. Cependant, certaines cellules, dont font partie les synoviocytes, sont réfractaires à la transduction par un vecteur adénoviral. Aucun vecteur permettant une transduction efficace des synoviocytes pour délivrer le gène codant pour la protéine PUMA à ces cellules n'a à ce jour été décrit.

Plusieurs études ont montré que le statut réfractaire de certaines cellules à une transduction par adénovirus tenait principalement à l'absence de la glycoprotéine CAR (pour « *coxsackie-adenovirus receptor* ») à la surface des cellules primaires humaines ou des lignées cellulaires continues (Gaden et al., Am. J. Respir. Cell Mol. Biol., 27 :628-40, 2002), ou à la faible accessibilité de CAR au pôle apical de l'épithélium humain (Granio et al., Hum. Gene Ther., 21 :1-19, 2010 ; Walters et al., J. Biol. Chem., 274 :10219-226, 1999). CAR est le récepteur cellulaire de haute affinité pour le vecteur adénoviral de type 5, HAdV5 (Bergelson et al., Science, 275 :1320-23, 1997 ; Tomko et al., Proc. Natl Acad. Sci. USA, 94 :3352-6, 1997), ainsi que pour d'autres sérotypes d'adénovirus (Arnberg, Rev. Med. Virol., 19 :165-78, 2009). HAdV5 est le sérotype d'adénovirus modèle, le plus utilisé pour la construction de vecteurs thérapeutiques. Différentes stratégies ont été développées pour rediriger les vecteurs HAdV5 vers d'autres récepteurs que CAR (Arnberg, 2009, précité ; Russell, J. Gen. Virol., 90 :1-20, 2009). La plupart de ces stratégies ont consisté en des modifications de la fibre de la particule virale d'adénovirus (Arnberg, 2009, précité ; Corjon et al., Mol. Ther., 16, 1813-24, 2008; Henning et al., Gene Ther., 12:211-24, 2005 ; Henning et al., Hum. Gene Ther., 13 :1427-39, 2002 ; Hong et al., Mol. Ther., 7 :692-9, 2003 ; Law et al., Mol. Ther., 12 :599-609, 2005 ; Magnusson et al., Cancer Gene Ther., 14 :468-79, 2007 ; Magnusson et al., J. Virol., 75 :7280-9, 2001 ; Magnusson et al., J. Gene Med., 4 :356-70, 2002 ; Russell, 2009, précité), la fibre étant la protéine de capside impliquée dans la reconnaissance des récepteurs de surface cellulaire par l'adénovirus. Cependant, certaines modifications génétiques du gène de la fibre d'adénovirus ont été identifiées comme étant préjudiciable à la viabilité ou au taux de croissance des vecteurs résultants (Henning et al., J. Gen. Virol., 87 :3151-60, 2006; Magnusson et al., 2002, précité). Dans d'autres cas, les modifications du vecteur ont seulement faiblement amélioré la transduction. Par exemple, des vecteurs HAdV5 modifiés génétiquement, conçus pour cibler les molécules d'intégrine à la surface des synoviocytes, n'ont abouti qu'à une amélioration modeste de l'efficacité de transduction de ces cellules *in vitro* et *ex vivo* (Toh et al., J. Immunol., 175(11) :7687-98, 2005).

D'autres stratégies proposées ont impliqué des modifications génétiques et chimiques de HAdV5 (Kreppel et al., Mol. Ther., 12 :107-117, 2005), ou le revêtement de la capside virale par un ligand peptidique ou protéique utilisé en tant qu'adaptateur bifonctionnel, à la fois pour le virus et pour la molécule de surface cellulaire spécifique (Hong et al., Hum. Gene Ther., 10 :2577-86, 1999 ; Dreier et al., J. Mol Biol., 405 :410-26, 2011).

Récemment, il a été rapporté que l'incorporation de la glycoprotéine CAR humaine (GenBank NP_001329) dans l'enveloppe d'un baculovirus produisait un baculovirus recombinant, nommé BV^{CAR}, capable de former avec HAdV5 un complexe stable, et d'augmenter significativement l'efficacité de transduction par HAdV5 de cellules réfractaires à cette transduction (Demande PCT WO 2011/042769 ; Granio et al., J. of Virology, 83(12) :6048-66, 2009).

Les Inventeurs ont testé si l'utilisation d'un baculovirus recombinant BV^{CAR} pouvait permettre d'augmenter l'efficacité de transduction des synoviocytes par un vecteur adénoviral portant le gène codant pour la protéine PUMA. Ils ont observé que le complexe HAdV5-BV^{CAR} ainsi formé permettait de transduire très efficacement des synoviocytes primaires, et d'induire leur apoptose. En outre, les Inventeurs ont constaté que, contrairement à ce qui avait été observé par Kammouni et al. avec la protéine *« BH3-only* » BNIP3, la présence de cytokines proinflammatoires n'inhibait pas l'effet pro-apoptotique de PUMA dans les synoviocytes, mais qu'au contraire, de manière surprenante, ces cytokines induisaient une augmentation de l'effet pro-apoptotique de PUMA, ce qui est particulièrement intéressant dans un contexte inflammatoire.

Les inventeurs ont également testé l'effet thérapeutique du complexe BV^{CAR}HAdV5-PUMA dans le modèle d'arthrite induite par adjuvant (AIA) chez le rat. Ils ont observé que l'injection intra-articulaire du complexe BV^{CAR}HAdV5-PUMA chez des rats arthritiques produisait une amélioration clinique, fonctionnelle et anatomique significative, dose-dépendante, au niveau des articulations traitées. Les inventeurs ont notamment montré que contrairement aux vecteurs conventionnels dérivés de l'HAdV5 qui ont une activité pro-inflammatoire, BV^{CAR}HAdV5-PUMA a une action globale anti-inflammatoire articulaire. Cette activité anti-inflammatoire articulaire est importante, comparée à la diminution discrète observée avec le vecteur HAdV5-PUMA. En outre, l'activité anti-inflammatoire articulaire importante de BV^{CAR}HAdV5-PUMA est détectée à des doses 10 fois inférieures (en termes de nombre de particules virales du vecteur actif HAdV5-PUMA) à celles utilisées avec le vecteur HAdV5-PUMA. Ces résultats démontrent le potentiel thérapeutique du complexe BV^{CAR}HAdV5-PUMA dans le traitement local des pathologies articulaires.

La présente invention a en conséquence pour objet un complexe constitué a) d'un adénovirus recombinant exprimant le gène codant pour la protéine PUMA sous contrôle d'un promoteur fonctionnel dans une cellule de mammifère et b) d'un baculovirus recombinant contenant un récepteur coxsackie-adénovirus (CAR) de mammifère inséré dans son enveloppe, pour l'utilisation comme médicament induisant l'apoptose des synoviocytes pour le traitement local d'une maladie articulaire inflammatoire liée à un défaut d'apoptose des synoviocytes, et caractérisé en ce que l'induction de l'apoptose des synoviocytes se produit en présence de cytokines pro-inflammatoires.

Comme expliqué ci-dessus, le complexe selon l'invention induit l'apoptose des synoviocytes en présence de cytokines proinflammatoires de façon très efficace, ce qui est particulièrement intéressant dans le traitement local des pathologies articulaires inflammatoires.

Le complexe selon l'invention peut en particulier être utilisé pour le traitement de l'inflammation articulaire due à la polyarthrite rhumatoïde ou à un autre rhumatisme inflammatoire, ou intervenant au cours des poussées d'arthrose, de la chondrocalcinose, de la goutte, de l'arthropathie hémophilique, du descellement de prothèses articulaires, ou d'une tumeur synoviale telle que rencontrée notamment dans le cas de la synovite villo-nodulaire.

Selon un mode de réalisation préféré de l'invention, ladite maladie articulaire est la polyarthrite rhumatoïde.

Le complexe selon l'invention est administré à un individu par une voie adaptée au traitement local d'une maladie articulaire et à des doses suffisantes pour obtenir l'effet thérapeutique désiré, qui peuvent être déterminées aisément par l'Homme du métier.

Le complexe selon l'invention sera notamment administré par voie intra-articulaire ou péri-articulaire, par exemple par injection ou infiltration.

Les complexes adénovirus-BV^{CAR} utilisables pour exprimer la protéine PUMA dans le cadre de la présente invention sont ceux décrits dans la Demande PCT WO 2011/042769. La protéine PUMA est de préférence la protéine humaine. La séquence de la protéine PUMA humaine a été déterminée par Yu et al. (Mol. Cell., 7(3) :673-82, 2001) et par Nakano et al. (Mol. Ce//. ; 7(3) :683-94, 2001). La séquence de l'ADNc et de la protéine PUMA humaine est enregistrée dans GenBank, sous le numéro AF332558.

La présente invention sera mieux comprise à l'aide du complément de description qui va suivre, qui se réfère à des exemples non-limitatifs de mise en oeuvre de l'invention, ainsi qu'aux dessins annexés, dans lesquels :

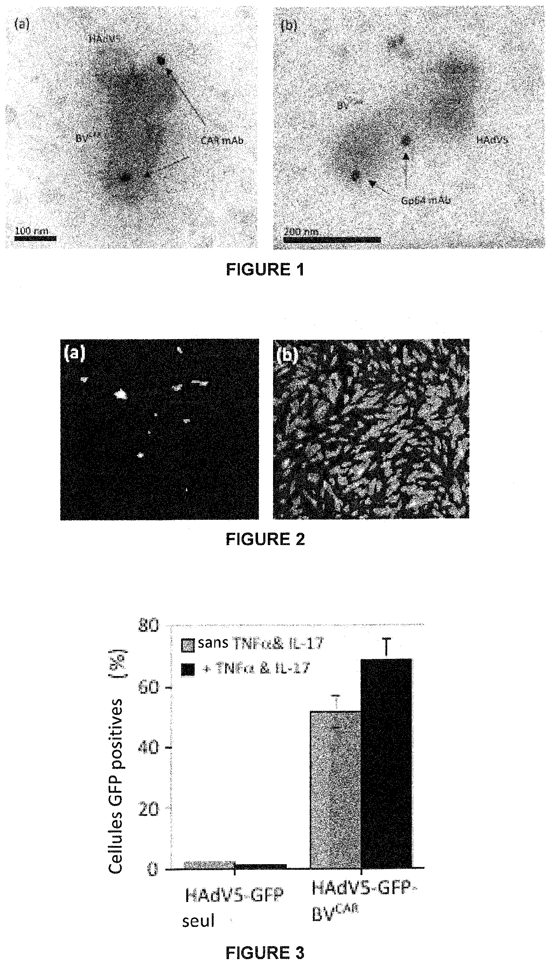

La **Figure 1** montre le complexe HadV5-GFP-BV^{CAR} observé en microscopie immuno-électronique. Le vecteur HadV5-GFP apparaît comme une particule icosaédrique de 80 nm de diamètre, lié à BV^{CAR} en forme de barre de 300-350 nm de longueur. Dans la **Figure 1a****,** les molécules CAR sont révélées par leur immunoréactivité avec un anticorps monoclonal anti-CAR (CAR mAb) et un anticorps secondaire marqué à l'or, apparaissant sous forme colloïdale de 20 nm. Dans la **Figure 1b****,** les glycoprotéines d'enveloppe gp64 du BV sont à leur tour marquées avec un anticorps anti-gp64 (Gp64 mAb) et un anticorps secondaire marqué à l'or.
La **Figure 2** illustre la transduction de synoviocytes humains primaires, observée par microscopie à fluorescence, par un vecteur HAdV5-GFP seul (**Figure 2a**) ou par le complexe HAdV5-GFP-BV^{CAR} (**Figure 2b**).
La **Figure 3** montre le pourcentage des cellules positives à GFP, quantifiées par cytométrie de flux, dans les échantillons de synoviocytes des Figures 2a et b, soit non prétraitées (w/o TNFα & IL-17), soit prétraitées avec les cytokines pro-inflammatoires TNF-α et IL-17 (+ TNFα & IL-17).

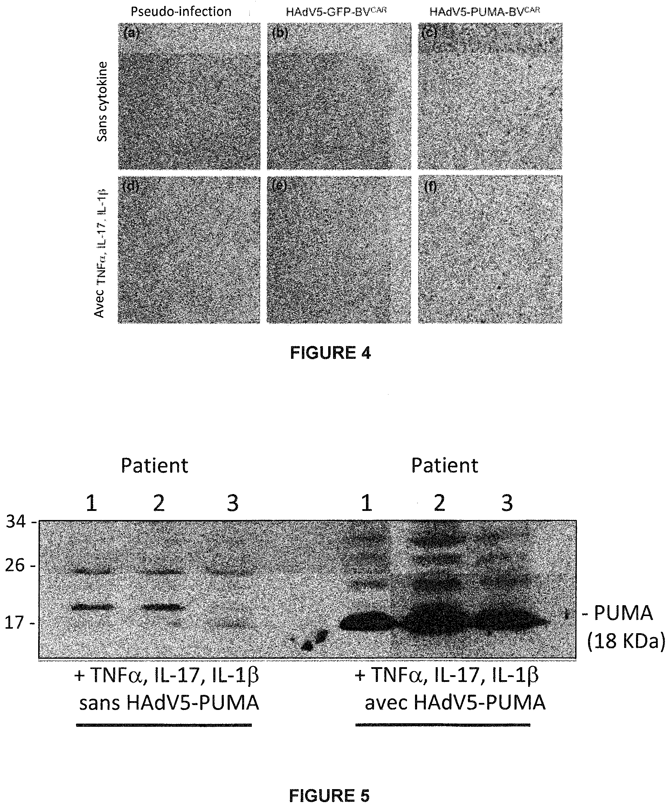

La **Figure 4** illustre l'effet de l'expression de la protéine PUMA sur les synoviocytes humains, observés au microscope. Les 3 photos de la ligne du dessus (a, b, c) correspondent aux cultures de synoviocytes non prétraités et celles de la ligne du dessous (d, e, f) aux synoviocytes prétraités avec les cytokines proinflammatoires TNF-α, IL-17 et IL-1β. La colonne de gauche (a et d) montre les cellules pseudo-infectées (« *mock-infected »* en anglais), c'est-à-dire ayant subi le protocole d'infection mais en l'absence de vecteur (0 vp/cell), la colonne du milieu les cellules infectées avec HAdV5-GFP-BV^{CAR} (b et e), et la colonne de droite les cellules infectées avec HAdV5-PUMA-BV^{CAR} (c et f).
La **Figure 5** montre l'expression de la protéine PUMA dans plusieurs échantillons de synoviocytes humains (échantillons issus des patients 1, 2 et 3) prétraités par le TNF-α, l'IL-17 et l'IL-1β, soit non-infectés (échantillons témoins, trois pistes de gauche), soit infectés par le complexe viral HAdV5-PUMA-BV^{CAR} (trois pistes de droite). La protéine PUMA (18 kDa) présente dans les lysats cellulaires a été mise en évidence par électrophorèse en gel de polyacrylamide-SDS suivi d'immunotransfert et révélation par un anticorps spécifique anti-PUMA.

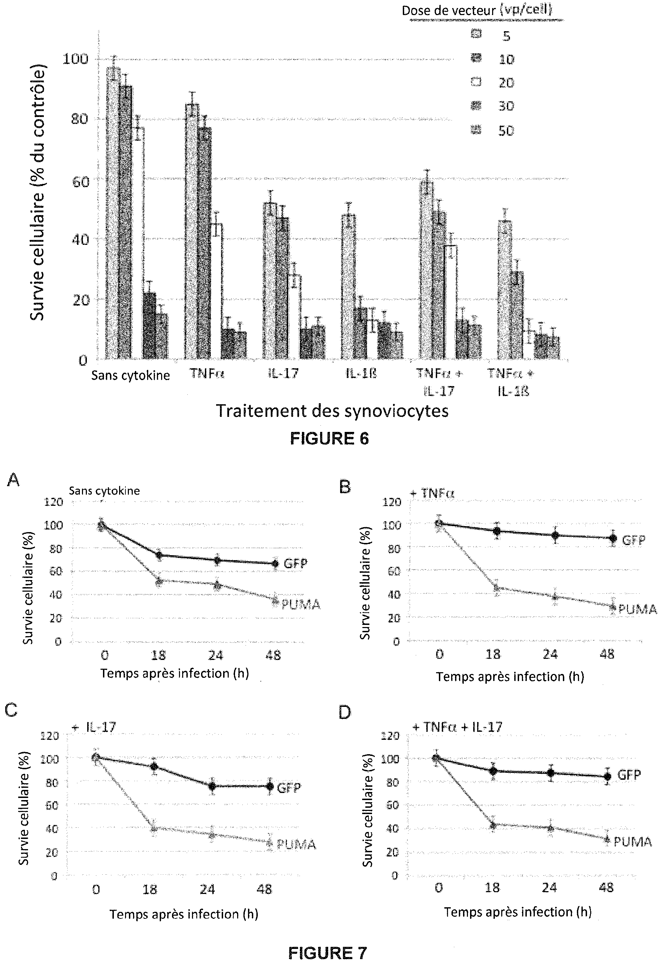

La **Figure 6** montre la dépendance à la dose de vecteurs de la mort cellulaire induite par PUMA. Des synoviocytes humains non traités et traités avec les cytokines TNF-α, IL-17 et IL-1β, ou en combinaison TNF-α + IL-17 et TNF-α + IL-1β, ont été infectées à des doses croissantes de HAdV5-PUMA-BV^{CAR}. La mort cellulaire a été évaluée en utilisant un test MTT, et le nombre de cellules survivantes a été exprimé comme le pourcentage par rapport aux cellules contrôle, pseudo-infectées (0 vp/cell), dont le nombre définit la valeur 100%.
La **Figure 7** illustre la cinétique de mort cellulaire due à l'expression de PUMA. Des synoviocytes non traités (A) ou traités avec TNF-α (B), IL-17 (C), ou un mélange de TNF-α et IL-17 (D), ont été infectés à une dose de vecteur constante de 20 vp/cell, la viabilité cellulaire a été suivie pendant 48h. HAdVS-GFP-BV^{CAR} a été utilisé à la même dose de vecteur en tant que contrôle. La mort cellulaire a été évaluée par test MTT et le nombre de cellules survivantes exprimé comme le pourcentage par rapport au nombre de cellules contrôle pris à temps 0 d'infection, auquel on attribue la valeur 100%.

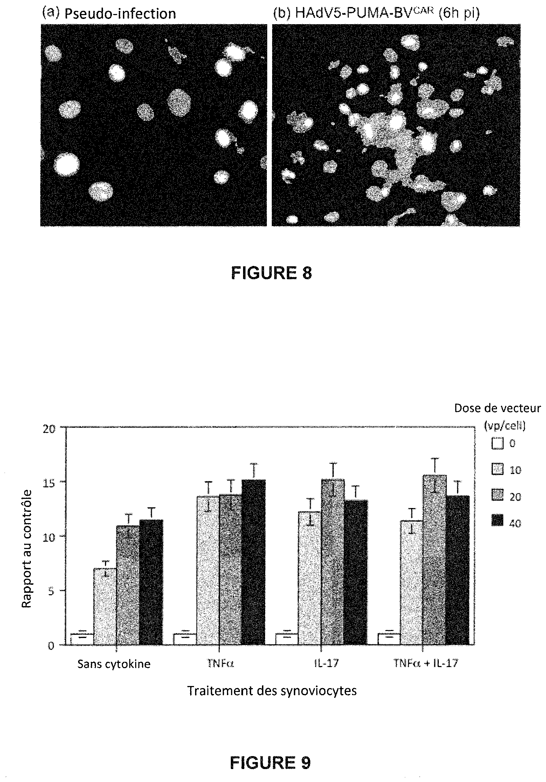

La **Figure 8** montre la fragmentation nucléaire des synoviocytes humains infectés avec HAdV5-PUMA-BV^{CAR}. Microscopie à fluorescence des synoviocytes pseudo-infectés (0 vp/cell) (a) et des synoviocytes infectés par HAdV5-PUMA-BV^{CAR} à 50 vp/cell (b), et récoltés 6h après infection (pi).
La **Figure 9** illustre la quantification de la libération cytoplasmique des nucléosomes dans les synoviocytes humains infectés avec HAdV5-PUMA-BV^{CAR}. Des synoviocytes, traités ou non avec des cytokines (TNF-α seul, IL-17 seul, ou TNF-α et IL-17 en mélange), ont été infectés à des doses croissantes de HAdV5-PUMA-BV^{CAR} et récoltés 24h après infection. La fragmentation de l'ADN et la libération des nucléosomes dans le cytoplasme ont été déterminés en utilisant la détection immunologique des fragments d'ADN complexés aux histones. Les lysats des cellules pseudo-infectées (0 vp/cell) ont servi de contrôle négatif pour évaluer le niveau basal, c'est-à-dire le contenu physiologique du cytoplasme en nucléosomes. Les résultats ont été exprimés en multiples du niveau basal, auquel a été attribuée la valeur 1.

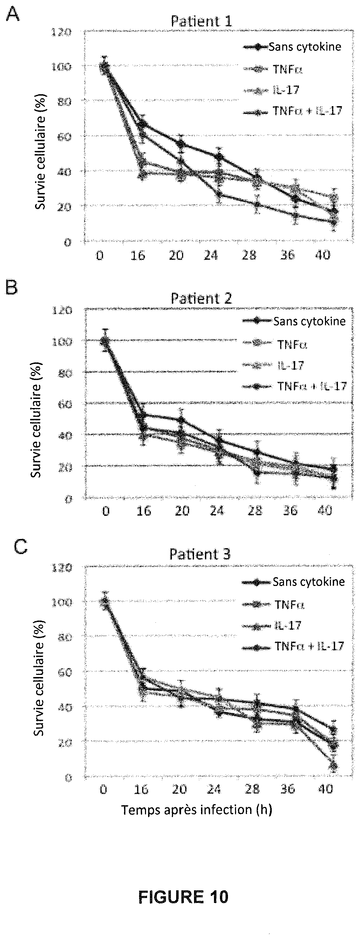

La **Figure 10** représente les résultats de l'infection de synoviocytes obtenus à partir d'échantillons de 3 patients, par HadV5-PUMA-BV^{CAR} (A : Patient 1 ; B : Patient 2 ; C : Patient 3). Ces synoviocytes, traités ou non par des cytokines (TNF-α seul, IL-17 seul, ou TNF-α et IL-17 en mélange), ont été infectés avec HAdV5-PUMA-BV^{CAR} à une dose constante de vecteur adénoviral de 50 vp/cell. La viabilité cellulaire a été suivie pendant 40h, en utilisant le test MTT. Le nombre de cellules survivantes a été exprimé comme le pourcentage de cellules contrôle, pris au temps 0 de l'infection, auquel a été attribuée la valeur 100%.

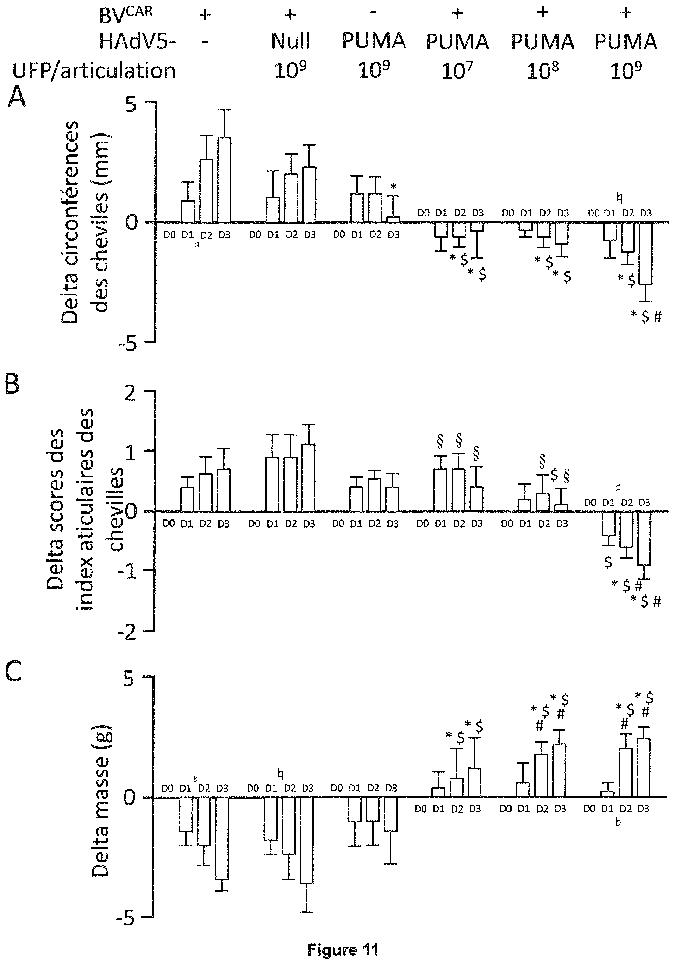

La **Figure 11** illustre la réduction de l'inflammation articulaire médiée par BV^{CAR}HAdV5-PUMA dans le modèle AIA chez le rat.

Les rats développent une arthrite 8-10 après l'injection d'adjuvant. A l'apparition de l'arthrite, 14 jours après l'injection, les vecteurs (10 µL) ont été délivrés dans l'articulation de chaque cheville. Trente rats divisés en 6 groupes (5 rats par groupe) ont été utilisés. Les trois groupes contrôle étaient constitués du baculovirus recombinant seul (BV^{CAR}; 10⁵ UFP par articulation), du vecteur adénovirus portant le gène PUMA seul (HAdV5-PUMA; 10⁹ UFP par articulation), et du BV^{CAR} (10⁵ UFP par articulation) en combinaison avec le vecteur adénoviral vide (HAdV5-null; 10⁹ UFP par articulation). Les trois groupes thérapeutiques étaient constitués de l'association de BV^{CAR} (10⁵ UFP par articulation) avec HAdV5-PUMA à 3 concentrations (10⁷, 10⁸ , et 10⁹ UFP par articulation, respectivement). Les valeurs représentées dans le graphe en barres sont les moyennes ± SEM. Les différences (Delta) dans les paramètres biologiques ont été définies par les valeurs obtenues le jour du suivi moins les valeurs obtenues le jour de l'injection intra-articulaire. (A), Différences dans les circonférences des chevilles. Les valeurs des circonférences des chevilles ont été obtenues par des mesures du diamètre des chevilles avec un pied-à-coulisse perpendiculaire, à l'aide d'une formule géométrique **(B)** Différences dans les scores des index articulaires des chevilles. **(C),** Différences dans les masses corporelles des rats. *Abréviations:* D, jour après l'injection intra-articulaire du vecteur; *, *P* < 0.05 versus BV^{CAR} seul; $, *P* < 0.05 versus BV^{CAR}HAdVS-null; #, *P* < 0.05 versus HAdV5-PUMA seul; §, *P* < 0.05 versus BV^{CAR}HAdV5-PUMA (10⁹ UFP par articulation); 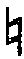 , *P* < 0.05 par le test ANOVA à un critère.

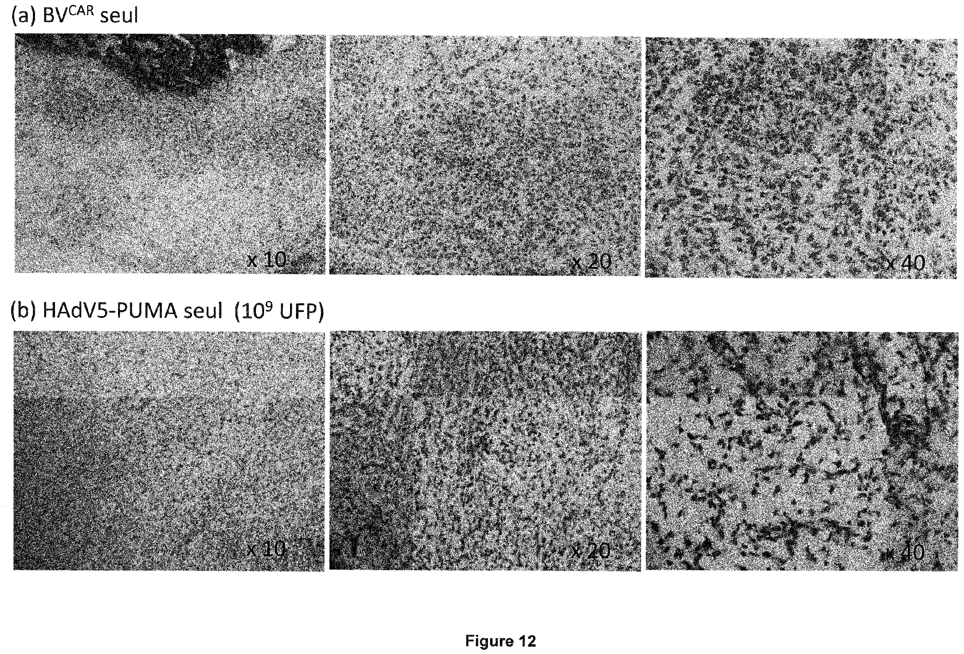

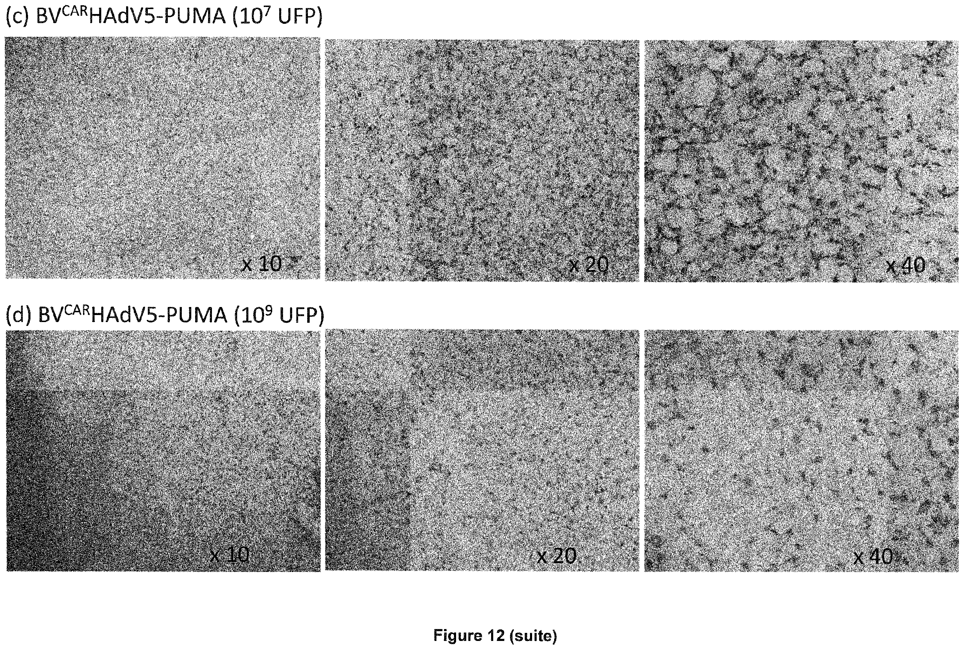

La **Figure 12** illustre l'analyse histologique des articulations injectées avec les vecteurs contrôle BV^{CAR} et HAdV5-PUMA, par rapport au vecteur duo BV^{CAR}HAdV5-PUMA. Les coupes fraîchement congelées (10 µm d'épaisseur) des articulations des chevilles des trois groupes de rats recevant **(a)** BV^{CAR} seul, **(b)** HAdV5-PUMA seul, ou **(c, d)** duo BV^{CAR}HAdV5-PUMA à différentes doses de vecteur HAdV5-PUMA, 10⁷ UFP **(c),** et 10⁹ par articulation **(d),** respectivement, ont été colorées avec H&E et la présence d'infiltrats des tissus synoviaux a été examinée sous un microscope optique. Des photographies représentatives de chaque groupe sont présentées à plusieurs grandissements: x10, panneau de gauche; x20, panneau central; et x40, panneau de droite.

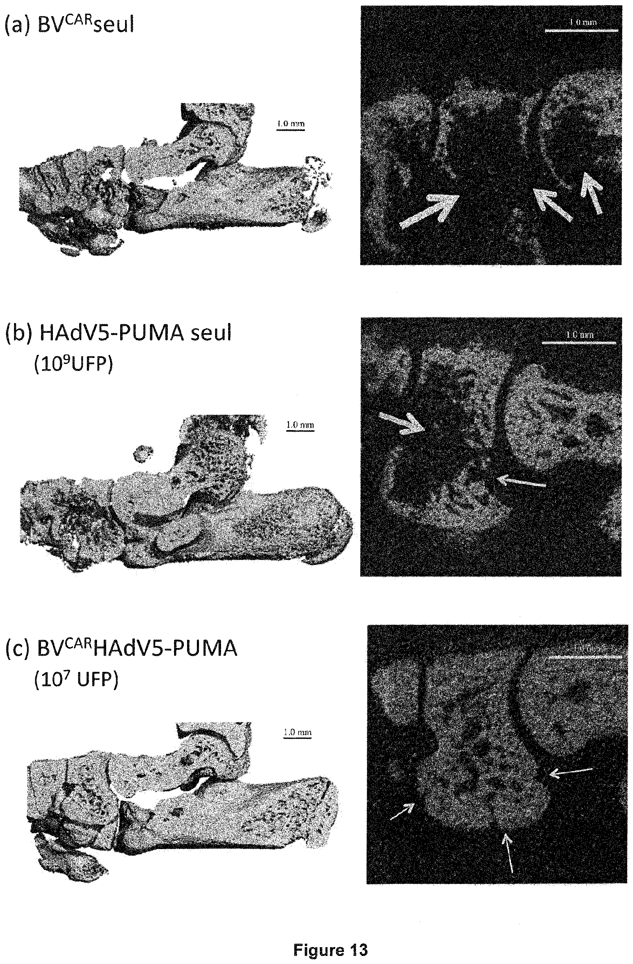

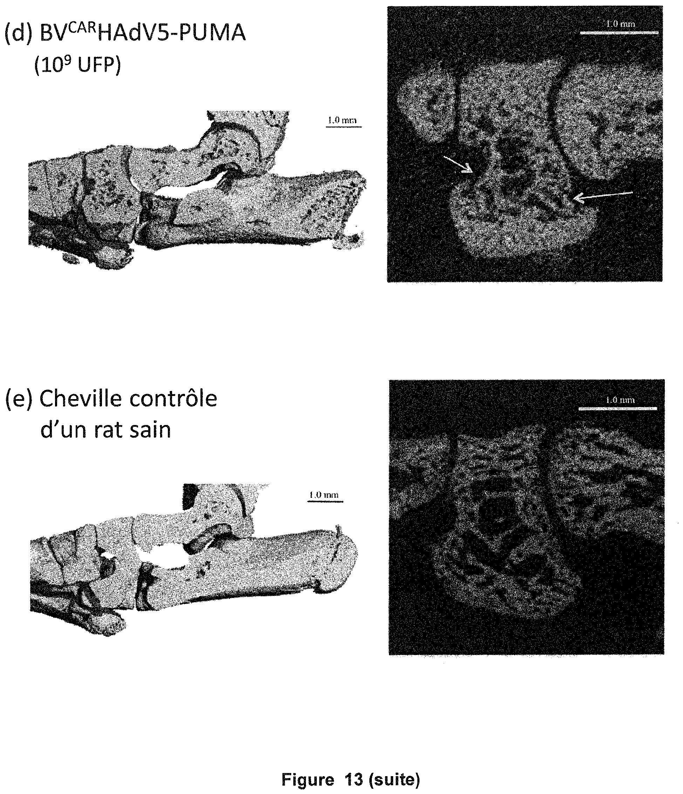

La **Figure 13** illustre l'imagerie des tissus des chevilles des rats par micro-tomographie informatisée **(µ-CT).** Les chevilles des rats ont été injectées avec **(a)** BV^{CAR} seul, **(b)** HAdV5-PUMA seul, ou **(c, d)** duo BV^{CAR} HAdV5-PUMA à différentes doses de vecteur HAdV5-PUMA, 10⁷ UFP par articulation **(c),** et 10⁹ **(d),** respectivement. **(e),** Cheville contrôle négatif d'un rat sain du même âge. ***Panneaux de gauche,*** vues parasagittales 3D de la totalité des chevilles de rat. ***Panneaux de droite,*** zoom sur des coupes parasagittales de µ-CT montrant de la gauche vers la droite, le cunéiforme intermédiaire, le naviculaire et le talus distal. Les flèches blanches indiquent les érosions des os.

### EXEMPLE 1 : TRANSDUCTION DE SYNOVIOCYTES PAR HAdV5-BV^{CAR}

Les synoviocytes utilisés dans les présents exemples ont été obtenus à partir de tissu synovial de patients atteints de polyarthrite rhumatoïde (PR) subissant une chirurgie de l'articulation et qui remplissaient les nouveaux critères de définition de la PR, établis par le Collège américain de rhumatologie (Aletaha et al., Arthritis & Rheumatism, 2569-81, 2010), puis ont été préparés comme décrit dans la Demande PCT WO 2011/042769.

Des vecteurs HAdV5 non réplicatifs, exprimant la protéine verte fluorescente (GFP, pour *green fluorescent protein*), sous le contrôle du promoteur fort précoce-immédiat du cytomégalovirus (CMV), ont été obtenus comme décrit dans la Demande PCT WO 2011/042769 sauf que les cellules de rein d'embryon humain HEK-293, ou «cellules 293 » (ATCC n° CRL-1573), ont été préalablement maintenues en monocouches en milieu DMEM (Invitrogen) supplémenté de 10% de SVF (Invitrogen), pénicilline (100 U/ml) et streptomycine (100 mg/ml) à 37°C et 5% CO₂.

Le vecteur baculovirus (BV) recombinant exprimant la protéine CAR (BV^{CAR}) a été obtenu comme décrit dans la Demande PCT WO 2011/042769.

Les complexes de vecteurs adénovirus et baculovirus et les cellules pour les essais de transduction ont été préparés comme décrit dans la Demande PCT WO 2011/042769.

L'expression cellulaire de GFP a été observée 36h après infection en microscopie à fluorescence et quantifiée par cytométrie de flux. Plus précisément, les cellules transduites ont été observées directement à partir de la plaque de culture, en utilisant un microscope inversé Axiovert (Zeiss). Les images de fluorescence ont été prises avec un appareil photographique digital Axiovert (Zeiss) et analysé à l'aide du programme Axio Vision (Zeiss). Puis, pour l'analyse en cytométrie de flux, les cellules ont été fixées aux temps appropriés avec 2% de paraformaldéhyde dans du PBS une nuit à 4°C, puis rincées une fois avec du PBS et analysées pour la fluorescence GFP à l'aide d'un cytomètre FACSCanto™ II et du logiciel DIVA 6 (Becton Dickinson Biosciences).

Les complexes HAdV5-BV^{CAR} ont été observés par microscopie électronique à transmission avec des échantillons colorés négativement et marqués à l'aide d'anticorps couplés à des particules d'or (marquage « immunogold »). Des anticorps monoclonaux dirigés contre la glycoprotéine CAR humaine (clone E1.1, Hemmi et al., Hum. Gene Ther., 9 :2363-2373, 1998) et contre la glycoprotéine gp64 du baculovirus (clone AcV1, Santa Cruz Biotechnology, Inc.) ont été utilisés pour marquer respectivement la présence de molécules CAR extrinsèques sur l'enveloppe de BV^{CAR} (Figure 1a), et de la glycoprotéine d'enveloppe gp64 intrinsèque du baculovirus (Fig. 1b).

Lorsque les synoviocytes ont été infectés avec HAdV5-GFP seul à une dose de vecteurs de 20 particules virales par cellule (vp/cell), l'efficacité de transduction est faible, avec moins de 2% de cellules positives GFP (Figures 2a et 3). En revanche, lorsque la même dose de vecteur HAdV5-GFP a été complexée avec BV^{CAR}, on observe plus de 50% de cellules positives GFP (Figures 2b et 3). De manière surprenante, quand les synoviocytes ont été prétraités avec des cytokines proinflammatoires (TNF-α et IL-17) pour mimer les conditions inflammatoires dans les articulations touchées par l'arthrite, 70% des cellules infectées avec HAdV5-GFP-BV^{CAR} ont été positives GFP (Figure 3).

### EXEMPLE 2: EXPRESSION DE LA PROTEINE PUMA DANS DES SYNOVIOCYTES A L'AIDE DE HAdV5-PUMA ET MORT DES CELLULES

Un vecteur basé sur HAdV5, portant le gène PUMA sous le contrôle du promoteur CMV, et couplé au vecteur recombinant BV^{CAR}, a été construit, en suivant le protocole décrit dans l'Exemple 1, en remplaçant GFP par PUMA, le gène PUMA étant ainsi inséré à la place de la région génique E1 elle-même délétée du génome d'HAdV5. La fonctionnalité de ce vecteur HAdV5-GFP-BV^{CAR} a été évaluée *in vitro* dans des synoviocytes primaires humains.

### - Observation au microscope -

Les effets cytologiques de l'expression de la protéine PUMA sur les synoviocytes ont d'abord été étudiés en comparant les synoviocytes infectés par HAdV5-PUMA et le vecteur contrôle HAdV5-GFP. Les deux vecteurs ont été utilisés à la même dose (50 vp/cell), et complexés à BV^{CAR}. L'aspect des cellules en monocouche a été observé, 24 h après infection, au microscope (Figure 4). Il n'y a pas eu de changement dans la morphologie des synoviocytes pseudo-infectés (Figure 4a) ou infectés avec HAdV5-GFP-BV^{CAR} (Figure 4b). L'infection avec HAdV5-PUMA-BV^{CAR} a montré une mort massive des cellules (Figure 4c). Ces résultats confirment que l'induction de la mort cellulaire des synoviocytes est due à la protéine PUMA.

Pour mimer les conditions inflammatoires rencontrées dans les articulations atteintes d'arthrite, la même expérience a été faite sur des synoviocytes préalablement traités avec 3 cytokines proinflammatoires différentes : TNF-α, IL-17 et IL-1β. Une mort cellulaire massive a eu lieu dans les synoviocytes infectés avec HAdV5-PUMA-BV^{CAR} (Figure 4f), alors qu'aucun changement visible n'a pu être observé dans les cellules pseudo-infectées (Fig. 4d) et les cellules infectées avec HAdV5-GFP-BV^{CAR} (Fig. 4e).

### - Évaluation du niveau d'expression de PUMA -

Trois isolats différents de synoviocytes humains prétraités par le TNF-α, l'IL-17 et l'IL-1β ont été infectés ou non (témoins non-infectés) par le complexe HAdV5-PUMA-BV^{CAR} afin d'analyser le niveau d'expression de la protéine PUMA. Les cellules ont été récoltées après 24h d'infection et les lysats cellulaires analysés par électrophorèse en gel de polyacrylamide-SDS et immunotransfert ('*SDS-PAGE* & *Western blotting').* Pour mettre en évidence la protéine PUMA, ont été utilisés successivement : (1) un anticorps polyclonal de lapin dirigé contre la protéine PUMA humaine (obtenu de la firme Epitomics), suivi (2) d'un anticorps anti-IgG de lapin marqué à la peroxydase (obtenu de la firme Sigma) ; (3) la réaction enzymatique de la peroxydase à été visualisée grâce à un substrat chimioluminescent (kit West-Pico, commercialisé par la firme Pierce). Les échantillons de cellules témoins non-infectées montrent un niveau très faible d'expression de la protéine PUMA dans les synoviocytes humains (expression dite 'basale'). En revanche, dans les échantillons de cellules infectées par le complexe HAdV5-PUMA-BV^{CAR}, on constate un niveau d'expression significativement élevé de la protéine PUMA, visible comme une bande majoritaire migrant à la position attendue pour une protéine de masse moléculaire de 18,000 daltons (18 kDa ; Fig. 5).

### - Mesure de la survie cellulaire à différentes doses de vecteur -

Les résultats d'observation au microscope ci-dessus montrent que l'utilisation de HAdV5-PUMA-BV^{CAR} à une dose de vecteur de 50 vp/cell a entraîné la mort de la quasi-totalité des synoviocytes en monocouches, 24h après infection. L'efficacité de la mort cellulaire induite par PUMA a été déterminée pour plusieurs doses de vecteurs, par numération des cellules vivantes par test MTT. Pour cela, des cellules cultivées sur plaques 96 puits à fond plat ont été infectées, et analysées 36 heures après infection. Le milieu de culture cellulaire a ensuite été retiré, et 30 µL de solution MTT (7,5 mg/ml de bromure de thiazolyl-tétrazolium (Sigma-Aldrich) dans du PBS) ont été ajoutés dans chaque puits et le tout a été incubé à 37°C pendant 4h. La solution MTT a alors été enlevée et 100 µL de DMSO (diméthylsulfoxyde, Sigma-Aldrich) ont été ajoutés dans chaque puits. La densité optique des surnageants dans la plaque 96 puits a été lue à 570 nm.

Les résultats sont indiqués à la Figure 6 et sont exprimés en pourcentage de survie cellulaire par rapport au contrôle. Pour les synoviocytes non traités aux cytokines, le pourcentage de cellules viables a diminué de manière dépendante à la dose de vecteur, avec 90% de cellules viables à 5 vp/cell, 75% à 20 vp/cell, et 15% à 50 vp/cell. Les synoviocytes infectés par HAdV5-PUMA-BV^{CAR} et traités avec TNF-α ont montré une baisse similaire de la viabilité cellulaire, de 83% de survie cellulaire à 5 vp/vell à environ 10% à 50 vp/cell. De manière surprenante, les synoviocytes prétraités à l'IL-17 ou l'IL-1β ont montré une susceptibilité significativement plus importante à PUMA à des faibles doses de vecteurs (5 à 20 vp/cell). Le traitement des synoviocytes avec TNF-α en combinaison avec IL-17 ou IL-1β a résulté en une plus grande sensibilité à la mort cellulaire induite par PUMA, comme observé avec les traitements avec IL-17 seul ou IL-1β seul. Ces résultats démontrent que le niveau de mort cellulaire induit par PUMA est dépendant de la dose de vecteur, et que les synoviocytes traités avec des cytokines proinflammatoires sont plus sensibles à la mort cellulaire induite par PUMA.

### EXEMPLE 3: CINETIQUES DE MORT CELLULAIRE APRES INFECTION AVEC HAdV5-PUMA-BV^{CAR}

Les cinétiques de mort cellulaire induite par PUMA des synoviocytes traités ou non par des cytokines ont été déterminées en mesurant le pourcentage de survie cellulaire aux temps 0, 18, 24 et 48h après infection, en utilisant une dose constante de HAdV5-PUMA-BV^{CAR} de 20 vp/cell, et analysées par test MTT, de la même manière que dans l'Exemple 2.

L'effet cytotoxique basal causé par l'infection avec un vecteur non réplicatif HAdV5 a été déterminé en utilisant HAdV5-GFP-BV^{CAR} à la même dose de vecteur que HAdV5-PUMA-BV^{CAR}. 18h après infection, environ 25% de la mort cellulaire était ainsi due à l'infection par HAdV5, indépendamment de l'effet de PUMA (Figure 7A). Entre 18 et 48h après infection, le pourcentage de cellules viables est resté stable, à environ 70%. L'infection avec HAdV5-PUMA a conduit à une baisse rapide de la proportion des cellules viables, avec 50% de survie cellulaire 18h après infection, et 30% 48h après infection.

Le prétraitement des synoviocytes avec TNF-α, IL-17 et la combinaison des deux cytokines a augmenté l'effet sur la mort cellulaire par la protéine PUMA, en obtenant seulement 40% de survie cellulaire 18h après infection contre 90% pour les synoviocytes infectés avec le vecteur contrôle HAdV5-GFP-BV^{CAR} (Figures 7B à D). Entre 18 et 48h, le pourcentage de survie cellulaire est resté stable à environ 80% pour les cellules infectées par le contrôle HAdV5-GFP-BV^{CAR}, alors qu'il a progressivement diminué jusqu'à 30% pour les cellules infectées par HAdV5-PUMA-BV^{CAR} . Ces résultats sont en accord avec les observations faites au microscope (Figure 4), et confirment que PUMA est directement impliquée dans l'induction de la mort cellulaire rapide des synoviocytes.

### EXEMPLE 4: FRAGMENTATION NUCLEAIRE ET APOPTOSE DES SYNOVIOCYTES INFECTES PAR HAdV5-PUMA-BV^{CAR}

L'observation en microscopie à fluorescence des synoviocytes incubés avec le colorant DAPI (4'-6'-diamidino-2-phénylindole) a montré une fragmentation de l'ADN dans les cellules infectées avec HAdV5-PUMA-BV^{CAR}. Une étude cinétique a indiqué que cette fragmentation nucléaire se produisait dès 6h après infection, alors que le nucleus des cellules contrôle, pseudo-infectées, est resté intact et entier (Figures 8a et b).

L'induction de la fragmentation de l'ADN et de la mort cellulaire par apoptose dans les cellules infectées par HAdV5-PUMA-BV^{CAR} a été évaluée quantitativement 24h après infection, par quantification des nucléosomes à l'aide d'un kit ELISA (kit « *Cell Death Detection* », Roche Diagnostics). Pour cela, des aliquotes de synoviocytes (10⁵ cellules) ont été récoltées 24h après infection, et centrifugées pendant 5 minutes à 200 g. Les culots cellulaires ont ensuite été resuspendus dans 250 µl de tampon d'incubation et incubés pendant 30 minutes à température ambiante. Les lysats cellulaires ont été centrifugés à 20000 g pendant 10 minutes et les surnageants ont été utilisés pour les mesures d'apoptose. Pour cela, un dixième de chaque surnageant a été incubé sur une plaque 96 puits préalablement revêtue d'anticorps anti-histone (clone H11-4), marqués à la biotine, pendant 90 minutes à température ambiante. Les puits ont été rincés 3 fois, et 100 µl de solution contenant un anticorps anti-ADN (clone M-CA-33) conjugué à la peroxydase ont été ajoutés dans chaque puits. La plaque 96 puits a ensuite été incubée à nouveau pendant 90 minutes à température ambiante. Les puits ont été à nouveau rincés 3 fois avant l'ajout de 100 µl de solution de substrat (ABTS) dans chaque puits. Enfin, la plaque a été incubée sur un mélangeur (à 250 rpm) pendant environ 20 minutes, et la densité optique (DO) a été mesurée à 405 nm.

Ce test a déterminé immunochimiquement les fragments d'ADN complexés aux histones qui ont été libérés dans la fraction cytoplasmique des lysats obtenus à partir des synoviocytes infectés avec HAdV5-PUMA-BV^{CAR} à différentes doses de vecteur. Les lysats obtenus à partir des cellules pseudo-infectées ont servi de contrôles négatifs et ont fourni le niveau basal des nucléosomes libérés physiologiquement dans le cytoplasme. En l'absence de cytokines, l'induction de l'apoptose par PUMA a été dose-dépendante, avec une libération de nucléosomes cytoplasmiques 6 fois plus importante que le niveau basal, à la dose de vecteur de 10 particules virales par cellule (vp/cell), et 12 fois plus importante à 20-40 vp/cell (Figure 9). En présence de TNF-α, d'IL-17, ou de ces 2 cytokines (TNF-α + IL-17), le niveau des nucléosomes cytoplasmiques a augmenté significativement, jusqu'à 12 à 15 fois le niveau basal à la dose de 10 vp/cell, et est resté à cette valeur plateau pour les plus fortes doses.

### EXEMPLE 5: EFFET DE HAdV5-PUMA-BV^{CAR} SUR LES SYNOVIOCYTES DERIVES D'ECHANTILLONS DE TISSU

Des synoviocytes primaires dérivés de 3 patients différents ont été utilisés pour évaluer de manière comparative leur sensibilité à l'apoptose induite par PUMA. L'infection par HAdV5-PUMA-BV^{CAR} a été réalisée à une dose de vecteur de 30 vp/cell, en l'absence ou en présence de cytokines proinflammatoires, et le niveau de cytotoxicité a été mesuré par test MTT, de manière similaire à l'Exemple 2, sur une période de 40h après infection. Les courbes de survie cellulaire des trois échantillons cliniques différents, représentées à la Figure 10, présentent des profils relativement similaires, avec une baisse rapide dans la survie cellulaire 16h après infection, suivie par une baisse plus progressive jusqu'à 40h. L'infection par HAdV5-PUMA-BV^{CAR} des synoviocytes primaires des patients 1 et 2 a montré un effet sur la mort cellulaire significativement plus important en présence des cytokines, en particulier d'IL-17, par rapport à l'effet observé en l'absence de cytokines (Figures 10A et B). Pour les synoviocytes du patient 3, il n'y a eu aucune différence dans l'induction de la mort cellulaire entre les cellules traitées ou non par les cytokines (Figure 10C). Dans les trois cas, le pourcentage de survie cellulaire 40h après infection a été réduit de manière drastique, jusqu'à 30% ou moins. Ces résultats démontrent que l'apoptose induite par HAdV5-PUMA-BV^{CAR} est efficace et rapide pour les synoviocytes dérivés de patients différents.

### EXEMPLE 6 : EFFET DE HAdV5-PUMA-BV^{CAR} DANS UN MODELE ANIMAL DE L'ARTHRITE

L'effet thérapeutique de HAdV5-PUMA-BV^{CAR} a été évalué dans le modèle de l'arthrite induite par adjuvant (AIA) chez le rat.

### Protocole expérimental

Des rats Lewis femelles d'environ 100g (Laboratoires Janvier, Saint-Berthevin, France) ont reçu une injection de 300 µL (5 mg/mL) de *Mycobacterium butyricum* lyophilisé (Difco Laboratories, Detroit, MI, USA; Marotte et al., Rheumatology, 2010, 49, 467-479), en sous-cutanée, à la base de la queue. Dans ce modèle, les premiers signes d'inflammation et de douleur articulaire apparaissent à partir de J8 après l'induction et atteignent un maximum entre J14 et J18 (Marotte et al., Rheumatology, 2010, 49, 467-479). Quatorze jours (J14) après l'induction, des vecteurs variés ont été injectés selon les 6 groupes suivants (5 rats par groupe). Les trois groupes contrôles étaient le baculovirus recombinant seul (BV^{CAR}; 10⁵ UFP par articulation), le vecteur adénovirus portant le gène PUMA seul (HAdV5-PUMA; 10⁹ UFP par articulation), et BV^{CAR} (10⁵ UFP par articulation) en combinaison avec un vecteur adénoviral vide (HAdV5-null; 10⁹ UFP par articulation). Les trois groupes thérapeutiques consistaient en l'association de BV^{CAR} (10⁵ UFP par articulation) avec HAdV5-PUMA à 3 concentrations (10⁷, 10⁸, and 10⁹ UFP par articulation, respectivement). Les paramètres cliniques ont été évalués tous les jours après l'injection intra-articulaire, pendant 4 jours, et incluaient un index articulaire, la circonférence de la cheville et la masse corporelle. Tous les animaux ont été euthanasiés à J4 après l'injection intra-articulaire. Les scores des index articulaires ont été enregistrés pour chaque articulation postérieure, en aveugle, par un observateur cohérent puis les moyennes ont été calculées pour chaque animal. Les scores ont été exprimés sur une échelle de 0 à 4, sur laquelle 0 = aucun gonflement ou érythème, 1 = léger gonflement et/ou érythème, 2 = oedème faible à modéré, 3 = oedème marqué avec usage limité de l'articulation et 4 = oedème excessif avec rigidité de l'articulation. Les circonférences des chevilles ont été mesurées, en aveugle, par le même observateur, comme décrit précédemment (Marotte et al., Rheumatology, 2010, 49, 467-479).

Les chevilles droites des animaux euthanasiés ont été dépecées, prélevées, incluses dans Neg50 (Thermo Scientific Waltham, MA), puis congelées dans l'azote liquide. Les chevilles droites congelées ont été scannées par tomographie micro-informatisée (µ-CT; viva-CT40, Scanco, Brütisellen, Switzerland). Les reconstructions tridimensionnelles ont été segmentées en utilisant les paramètres suivants : sigma, 2,8; support, 2; seuil, 289.

Après l'acquisition en microtomographie, les coupes des articulations fraîchement congelées (10 µm d'épaisseur) des différents groupes ont été colorées à l'hématoxyline et l'éosine, et la présence d'infiltrats a été examinée.

### Diminution de l'inflammation articulaire par injection intra-articulaire de BV^{CAR}HAdV5-PUMA dans un modèle AIA chez le rat

Au vu des résultats encourageants obtenus *in vitro* et *ex vivo,* l'effet de BV^{CAR}HAdV5-PUMA *in vivo* a ensuite été exploré. Dans le modèle d'arthrite inflammatoire AIA, les rats développent une arthrite 8-10 jours après l'injection d'adjuvant Après l'apparition de l'arthrite, *i.e.* au jour 14 (D14) post-induction, les vecteurs ont été administrés par injection intra-articulaire dans la cheville. Trente rats divisés en 6 groupes (5 rats par groupe) ont été utilisés. Les trois groupes contrôle étaient constitués de : (i) BV^{CAR} seul (10⁵ UFP par articulation), (ii) HAdV5-PUMA seul (10⁹ UFP par articulation), et (iii) complexe BV^{CAR}HAdV5-null (10⁵ UFP BV^{CAR} + 10⁹ UFP HAdV5-null par articulation). Les trois groupes thérapeutiques étaient constitués de BV^{CAR} (10⁵ UFP par articulation) complexé avec HAdV5-PUMA à 3 doses différentes (10⁷, 10⁸, and 10⁹ UFP par articulation, respectivement).

L'administration des vecteurs contrôle BV^{CAR} ou HAdV5-PUMA seul, ou du complexe contrôle BV^{CAR}HAdV5-null, n'a produit aucun effet bénéfique détectable sur l'évolution de l'arthrite (**Figure 11**). Cependant, BV^{CAR}HAdV5-PUMA a significativement amélioré les paramètres de tous les rats. BV^{CAR}HAdV5-PUMA a montré une forte réduction de la variation de la circonférence des chevilles et cet effet s'est produit de manière dose-dépendante (**Figure 11A**). De même, BV^{CAR}HAdV5-PUMA a induit une diminution dose-dépendante du score de l'index articulaire, mais la différence avec les vecteurs contrôle pour ce paramètre était significative aux doses les plus élevées du vecteur (10⁹ UFP par articulation; **Figure 11B**). De plus, les masses corporelles des rats injectés avec BV^{CAR}HAdV5-PUMA étaient supérieures à celles des animaux contrôle pour toutes les doses de BV^{CAR}HAdV5-PUMA testées (**Figure 11C**).

### Analyse histologique des chevilles de rat

L'efficacité du traitement a été étudiée à la fois sur les tissus articulaires et osseux. Les altérations de la synovie ont été évaluées en microscopie optique sur les coupes d'articulation fraîchement congelées, après coloration standard avec l'hématoxyline et l'éosine (**Figure 12**). Les infiltrats étaient significativement réduits dans les articulations traitées avec BV^{CAR}HAdV5-PUMA, par comparaison avec les articulations traitées avec BV^{CAR} seul (**Figure 12b**) ou HAdV5-PUMA seul (**Figure 12b**). Cet effet est dose-dépendant avec une diminution du nombre d'infiltrats moins prononcée à 10⁷ UFP (**Figure 8c**) qu'à 10⁹ UFP de BV^{CAR}HAdV5-PUMA par articulation de cheville (**Figure 8d**).

### Imagerie du tissu de la cheville de rat par tomographie micro-informatisée haute résolution aux rayons X (µ-CT)

Les altérations du tissus osseux chez les rats ont été étudiées *ex vivo* en utilisant la µ-CT. Les images en trois dimensions ont permis une évaluation complète de la perte d'os dans les articulations, alors que les détails plus fins ont été détectés sur les coupes parasagittales. Des altérations importantes des tissus ont été observées dans les articulations des rats injectées avec BV^{CAR} seul (**Figure 13a****).** Une perte étendue d'os était visible dans les articulations des rats injectés avec HAdV5-PUMA seul (**Figure 13b**). Par contraste, dans les articulations injectées avec les complexes BV^{CAR}HAdV5-PUMA, on observe uniquement des changements morphologiques discrets (**Figure 13c****,** **13d**), par rapport aux articulations de cheville des rats sains (**Figure 13e****).** Le seul marqueur de perte osseuse perceptible dans les articulations traitées avec BV^{CAR}HAdV5-PUMA étaient des modifications de porosité, caractéristiques de la phase précoce de l'arthrite. Des changements supplémentaires de la microarchitecture, consécutifs à une inflammation étendue, n'ont pas été observés dans les articulations traitées avec BV^{CAR}HAdV5-PUMA.

### Conclusions

L'effet net du vecteur duo BV^{CAR}HAdV5-PUMA sur le contrôle de la prolifération des synoviocytes observé *in vitro* et *ex vivo* a incité les inventeurs à étudier les effets de l'expression du gène pro-apototique PUMA *in vivo.* Pour ce faire, les inventeurs ont utilisé BV^{CAR}HAdV5-PUMA dans le modèle d'AIA chez le rat, un modèle animal largement reconnu et cohérent de l'arthrite rhumatoïde chez l'homme.

Les résultats observés dans les chevilles des rats injectées avec BV^{CAR}HAdV5-PUMA, comparés aux vecteurs contrôle, confirment ceux obtenus *in vitro* et *ex vivo.* Tous les effets bénéfiques du vecteur duo BV^{CAR}HAdV5-PUMA se produisent de manière dose dépendante, et incluent (i) une amélioration significative de la fonctionnalité de l'articulation, (ii) une forte diminution de l'inflammation locale, et (iii) une absence ou un niveau minimum d'altérations de l'articulation et de perte osseuse, détectable en histopathologie et en µ-CT. Par comparaison, l'administration des vecteurs contrôle BV^{CAR} ou HAdV5-PUMA seul, ou du complexe contrôle BV^{CAR}HAdV5-null, n'a produit aucun effet bénéfique détectable sur l'évolution de l'arthrite

Contrairement aux vecteurs conventionnels dérivés de HAdV5 qui ont une activité pro-inflammatoire, BV^{CAR}HAdV5-PUMA a une action globale anti-inflammatoire articulaire. Cette activité anti-inflammatoire articulaire est importante, comparée à la diminution discrète observée avec le vecteur HAdV5-PUMA. En outre, l'activité anti-inflammatoire articulaire importante de BV^{CAR}HAdV5-PUMA est détectée à des doses 10 fois inférieures (en termes de nombre de particules virales du vecteur actif HAdV5-PUMA) à celles utilisées avec le vecteur HAdV5-PUMA.

L'ensemble de ces résultats démontrent le potentiel thérapeutique du complexe BV^{CAR}HAdV5-PUMA dans le traitement local des pathologies articulaires.

## Revendications

1. Complexe constitué a) d'un adénovirus recombinant exprimant le gène codant pour la protéine PUMA sous contrôle d'un promoteur fonctionnel dans une cellule de mammifère et b) d'un baculovirus recombinant contenant un récepteur coxsackie-adénovirus (CAR) de mammifère inséré dans son enveloppe, pour son utilisation dans le traitement local d'une maladie articulaire inflammatoire par induction de l'apoptose des synoviocytes, **caractérisé en ce que** l'induction de l'apoptose des synoviocytes se produit en présence de cytokines pro-inflammatoires.

2. Complexe pour son utilisation selon la revendication 1, **caractérisé en ce que** ladite maladie articulaire est choisie parmi la polyarthrite rhumatoïde, les rhumatisme inflammatoires, les poussées d'arthrose, la chondrocalcinose, la goutte, l'arthropathie; hémophilique, le descellement de prothèses articulaires, et les tumeurs synoviales.

3. Complexe pour son utilisation selon la revendication 1 ou 2, dans lequel la protéine PUMA est la protéine PUMA humaine.

4. Complexe pour son utilisation selon l'une quelconque des revendications 1 à 3, dans lequel l'adénovirus est l'adénovirus humain de sérotype 5 (HAdV5).

5. Complexe pour son utilisation selon l'une quelconque des revendications 1 à 4, dans lequel le baculovirus est un virus de la polyédrose nucléaire de groupe 1 choisi parmi le virus de la polyédrose nucléaire multiple *d'Autographa californica* (AcMNPV) et le virus de la polyédrose nucléaire *Bombyx mori* (BmNPV).

6. Complexe pour son utilisation selon l'une quelconque des revendications 1 à 5, dans lequel le récepteur CAR est le récepteur CAR humain.

## Patentansprüche

1. Komplex, gebildet a) von einem rekombinanten Adenovirus, der das Gen exprimiert, das für das PUMA-Protein codiert, unter der Kontrolle eines funktionellen Promoters in einer Säugetierzelle, und b) von einem rekombinanten Baculovirus, der einen Coxsackie-Adnovirus-Rezeptor (CAR) eines Säugetiers enthält, eingesetzt in seine Hülle, für seine Verwendung bei der Lokalbehandlung einer entzündlichen Gelenkerkrankung durch Induktion der Apoptose der Synoviocyten, **dadurch gekennzeichnet, dass** die Induktion der Apoptose der Synoviocyten bei Anwesenheit proinflammatorischer Cytokine erfolgt.

2. Komplex für seine Anwendung nach Anspruch 1, **dadurch gekennzeichnet, dass** die Gelenkerkrankung aus der rheumatischen Polyarthritis, den entzündlichen Rheumatismen, 'den Arthroseschüben, der Chondrocalcinose, der Gicht, der hämophilen Arthropathie, dem Lösen von Gelenkprothesen und den Synovialtumoren ausgewählt ist.

3. Komplex für seine Anwendung nach Anspruch 1 oder 2, wobei das PUMA-Protein das humane PUMA-Protein ist.

4. Komplex für seine Anwendung nach einem der Ansprüche 1 bis 3, wobei der Adenovirus der humane Adenovirus vom Serotyp 5 (HAdV5) ist.

5. Komplex für seine Anwendung nach einem der Ansprüche 1 bis 4, wobei der Baculovirus ein Nucleopolyhedrovirus der Gruppe 1 ist, ausgewählt aus dem Virus der multiplen Nucleopolyhedrose von *Autographa californica* (AcMNPV) und dem Nucleopolyhedrovirus *Bombyx mori* (BmNPV).

6. Komplex für seine Anwendung nach einem der Ansprüche 1 bis 5, wobei der CAR-Rezepter der humane CAR-Rezeptor ist.

## Claims

1. Complex consisting of a) a recombinant adenovirus expressing the gene coding for the PUMA protein under the control of a functional promoter in a mammalian cell, and b) a recombinant baculovirus containing a mammalian coxsackie adenovirus (CAR) receptor inserted into its envelope, for its use in the local treatment of an inflammatory articular disease by induction of the apoptosis of synoviocytes, **characterized in that** the induction of apoptosis of synoviocytes is produced in the presence of pro-inflammatory cytokines.

2. Complex for its use according to claim 1, **characterized in that** the said joint disease is selected from rheumatoid polyarthritis, inflammatory rheumatism, osteoarthritis attacks, chondrocalcinosis, gout, hemophilic arthropathy, loosening of prosthetic joints, and synovial tumors.

3. Complex for use according to claim 1 or 2, wherein PUMA protein is human PUMA protein.

4. Complex for use as claimed in any one of the claims 1 to 3, wherein the adenovirus is human serotype 5 adenovirus (HAdV5).

5. Complex for use as claimed in any one of the claims 1 to 4 wherein the baculovirus is a Group 1 nuclear polyhedrosis virus selected from autographa californica multiple nuclear polyhedrosis virus (AcMNPY) and the Bombyx mori nuclear polyhedrosis virus (BmNPV).

6. Complex for use according to any one of the claims 1 to 5, wherein the CAR receptor is the human CAR receptor.
